# EUROPEAN PATENT APPLICATION

(11) **EP 4 238 509 A1**
(43) Date of publication of application: **06.09.2023**
(21) Application number: 21902704.2
(22) Date of filing: 09.12.2021
(51) Int. Cl.: A61B 17/072

(54) **ELECTRIC STAPLER AND CONTROL METHOD THEREFOR**

(30) Priority: 11.12.2020 CN 202011448523
(71) Applicant: Fulbright Medical Inc., Wuxi, Jiangsu 214437 (CN)
(72) Inventor: SUN, Baofeng, Wuxi, Jiangsu 214437 (CN); SUN, Haimeng, Wuxi, Jiangsu 214437 (CN)
(74) Representative: Novagraaf Technologies
(86) International application number: PCT/CN2021/136915
(87) International publication number: WO 2022/121999

(57) **Abstract**

Disclosed are an electric stapler (100) and a control method therefor. The electric stapler includes: an operating handle (10); a shaft assembly (20); a cutting knife assembly (40), including a knife pushing member (42); a motor (80), provided in the operating handle (10); a trigger (60), including: a firing unit (51), configured to send a firing signal representing a knife feeding action and a firing stopping signal representing stopping of the knife feeding action; and a knife returning unit (63), configured to send a knife returning signal representing a knife returning action and a knife returning stopping signal representing stopping of the knife returning action; and a control module (90), electrically connected to the motor (80) and the trigger (60), where the knife pushing member (42) has, during the knife feeding action, a first state of stopping between an initial position and a final firing position, when in the first state, the control module (90), when detecting the knife returning signal, controls the knife pushing member (42) to perform the knife returning action towards the final firing position, and during the knife returning action, the control module (90), when detecting the knife returning stopping signal, controls the knife pushing member (42) to stop the knife returning action. The electric stapler (100) is able to control the knife returning action and prevent the knife returning member (42) from directly returning to the initial position, thereby improving the fault tolerance capability and the firing capacity.

## Description

### Cross-Reference to Related Application

This present invention claims the priority of Chinese Invention No. 202011448523.4, entitled "Electric Stapler and Control Method Therefor" filed in China Patent Office on December 11, 2020, the entire disclosure of which is incorporated in the present invention by reference.

### Technical Field

The present invention relates to the technical field of medical instruments, in particular to an electric stapler and a control method therefor.

### Background

As we all know, endoscopic cutter staplers have been widely used in surgeries in cavities such as abdominal cavities. A stapler includes a jaw assembly, a cutting knife assembly, and a staple cartridge assembly. In one operating cycle, after the jaw assembly of the stapler extends into a human body from a puncture hole in a human skin, firstly, the stapler is operated to close the jaw assembly to clamp a target tissue; then, the stapler is operated to drive the cutting knife assembly to move forward to push the staple cartridge assembly to discharge a staple to staple the target tissue, meanwhile, the cutting knife assembly cuts the target tissue, and after the target tissue is stapled and cut, the stapler is operated to drive the cutting knife assembly to return to an initial position; and finally, the jaw assembly is opened to separate from the target tissue, the jaw assembly of the stapler is taken out from the puncture hole, and then a new staple cartridge may be reloaded for the next operating cycle.

An electric stapler below is provided in the related art, where a driving force is provided by using a battery and a motor to move a jaw assembly and a cutting knife assembly, and a body of the electric stapler is usually equipped with a firing button and a forced knife returning button for a user to operate. According to a specification of a staple cartridge assembly, a cutting knife has a corresponding movement stroke. When the user operates the firing button for the first time, the cutting knife assembly performs a knife feeding movement from rear to front, and starts to staple and cut a tissue. If the firing button stops being operated halfway, the motor may be stopped, so that the cutting knife assembly stops at any intermediate point of the movement stroke. In this case, the next action may be to operate the forced knife returning button by the user, so that the cutting knife assembly starts to perform a knife returning movement backward until a final end is reached; and the next action may also be to operate the firing button again, so that the cutting knife assembly continues moving forward to perform the knife feeding movement. Alternatively, the user keeps operating a firing switch, the motor will automatically stop until the cutting knife assembly moves to a most front end of the movement stroke, and the cutting knife assembly stops at the most front end. In this case, once the user no longer operates the firing button, the motor will start to make the cutting knife assembly move backward to perform the knife returning movement until the final end is reached. The cutting knife assembly adopts directly returning to the initial position. This knife returning manner is a one-step knife returning.

Under actual working conditions, the cutting knife assembly includes a long sheet-shaped knife pushing member located inside a sleeve and a cutting knife located at a front end of the knife pushing member. In the event of cutting a heavy load, the knife pushing member of the cutting knife assembly is prone to bending on an inner wall of the sleeve during the knife feeding movement, and a larger frictional force is generated between the knife pushing member and the inner wall of the sleeve, so that the cutting knife at the front end cannot be pushed to continue moving forward, and thus the knife feeding action cannot continue being performed. Existing control methods for electric staplers are unable to solve this problem.

### SUMMARY

To solve at least one technical problem in the related art, the present invention provides an electric stapler and a control method therefor, which is able to control a knife returning action and prevent a knife returning member from directly returning to the initial position, thereby improving the fault tolerance capability and the firing capacity.

To achieve the above objective, the present invention provides the following technical solutions:
There is provided an electric stapler, including:
an operating handle;
a shaft assembly, extending from the operating handle;
a cutting knife assembly, provided at one end, away from the operating handle, of the shaft assembly and including a knife pushing member;
a motor, provided in the operating handle and configured to drive the knife pushing member to move;
a trigger, including: a firing unit for a user to trigger, the firing unit being configured to send a firing signal representing a knife feeding action and a firing stopping signal representing stopping of the knife feeding action; and a knife returning unit for the user to trigger, the knife returning unit being configured to send a knife returning signal representing a knife returning action and a knife returning stopping signal representing stopping of the knife returning action; and
a control module, electrically connected to the motor and the trigger, where the control module controls, when detecting the firing signal, the motor to drive the knife pushing member to perform the knife feeding action towards a final firing position, and the control module controls, when detecting the firing stopping signal, the knife pushing member to stop the knife feeding action; and
the knife pushing member has, during the knife feeding action, a first state of stopping between an initial position and the final firing position, when in the first state, the control module controls, when detecting the knife returning signal, the knife pushing member to perform the knife returning action towards the initial position, and during the knife returning action, the control module controls, when detecting the knife returning stopping signal, the knife pushing member to stop the knife returning action.

In an embodiment mode, the knife pushing member has a second state of performing the knife returning action in the first state and stopping between the initial position and the final firing position, and in both the first state and the second state, the firing unit is in an effective state, so that the control module controls, based on the detected firing signal, the knife pushing member to perform the knife feeding action.

In an embodiment mode, the cutting knife assembly further includes a cutting knife located in front of the knife pushing member towards the final firing position, and when the knife pushing member is in the first state, a first stop position is provided at an end, close to the cutting knife, of the knife pushing member; and when the knife pushing member is in the second state, a second stop position is provided at an end, close to the cutting knife, of the knife pushing member, and the second stop position is located behind the first stop position or coincides with the first stop position.

In an embodiment mode, when the knife pushing member is in the first state, the control module controls, when detecting the knife returning signal, the knife pushing member to perform the knife returning action towards the initial position, and when the knife pushing member stops the knife returning action and reaches the initial position, the control module controls the firing unit to switch to be in from an effective state to a failed state, so as to control the knife pushing member to no longer perform knife feeding.

In an embodiment mode, the trigger includes a first position detection unit configured to detect whether the knife pushing member is located in the initial position and to output a first in-place signal representing that the knife pushing member is located in the initial position, and the control module controls, based on the first in-place signal, the motor to stop.

In an embodiment mode, when the knife pushing member performs the knife feeding action and reaches the final firing position, the control module controls the firing unit to switch to be in from an effective state to a failed state, so as to control the knife pushing member to no longer perform knife feeding.

In an embodiment mode, when the knife pushing member performs the knife feeding action and reaches the final firing position, the control module controls, when detecting the knife returning signal, the knife pushing member to leave the final firing position and perform the knife returning action towards the initial position until the initial position is reached.

In an embodiment mode, when the knife pushing member performs the knife feeding action and reaches the final firing position, the control module controls, when detecting the knife returning signal, the knife pushing member to leave the final firing position and perform the knife returning action towards the initial position, and during the knife returning action, the control module controls, when detecting the knife returning stopping signal, the knife pushing member to stop the knife returning action.

In an embodiment mode, when the knife pushing member has a third state of performing the knife returning action after reaching the final firing position and stopping between the initial position and the final firing position, and in the third state, the control module controls the firing unit to be kept in a failed state, so as to control the knife pushing member to no longer perform knife feeding.

In an embodiment mode, the trigger includes a second position detection unit configured to detect whether the knife pushing member is located in the final firing position and to output a second in-place signal representing that the knife pushing member is located in the final firing position, and the control module controls, based on the second in-place signal, the motor to stop and controls the firing unit to switch to be in from the effective state to the failed state, and controls, during the knife returning action, the firing unit to be kept in the failed state.

In an embodiment mode, the electric stapler further includes a jaw assembly, where the jaw assembly includes a staple cartridge seat and a staple abutting seat, the staple abutting seat is able to be opened and closed relative to the staple cartridge seat, and the cutting knife in the knife pushing member is accommodated in a space between the staple cartridge seat and the staple abutting seat; the trigger further includes: a jaw closing unit for the user to trigger a jaw closing signal representing a jaw closing action; and a jaw opening unit for the user to trigger a jaw opening signal representing a jaw opening action; and the control module controls, when detecting the jaw closing signal, the jaw assembly to perform the jaw closing action, and the control module controls, when detecting the jaw opening signal, the jaw assembly to perform the jaw opening action.

In an embodiment mode, the jaw closing unit and the firing unit are integrated as a first button, and the knife returning unit and the jaw opening unit are integrated as a second button.

There is provided a control method for an electric stapler, where the electric stapler includes a cutting knife assembly and a motor, the cutting knife assembly includes a knife pushing member, and the motor is configured to drive the knife pushing member to move; and the control method includes:
controlling the motor to rotate along a first direction, so that the motor drives the knife pushing member to perform a knife feeding action towards a final firing position;
when the knife pushing member has not reached the final firing position, controlling the motor to rotate along a second direction that is opposite to the first direction, so that the motor drives the knife pushing member to perform a knife returning action towards an initial position; and
when the knife pushing member has not returned to the initial position, controlling the motor to rotate along the first direction, so that the motor drives the knife pushing member to continue performing the knife feeding action towards the final firing position.

The beneficial effects are as follows:
Compared with the related art, the electric stapler and the control method therefor in the present invention may trigger the knife returning signal through user operation to cause the knife pushing member to perform the knife returning action. The knife returning action may be controlled by the control module based on the knife returning signal and the knife returning stopping signal sent by the knife returning unit, thereby preventing the knife pushing member from directly returning to the initial position in the event of misoperation.

During normal use, when the knife pushing member is not fully fired, if the user accidentally triggers the knife returning unit to send the knife returning signal, the knife pushing member performs the knife returning action. In this case, if the knife returning stopping signal is sent by the knife returning unit, the knife pushing member stops the knife returning action and is able to be fired again, so that the remaining staple cartridge is able to continue being used up and the ideal results are achieved for a surgery.

In addition, when encountering a heavy load, the knife pushing member is prone to bending on an inner wall of a sleeve during knife feeding, and a larger frictional force is generated between the knife pushing member and the inner wall of the sleeve, making it impossible to continue performing the knife feeding action. By triggering the knife returning unit, the knife pushing member is able to recover to be in a straight state after returning for a distance, so that there is no friction between the knife pushing member and the inner wall of the sleeve; and then, by triggering the firing unit, the knife pushing member is able to quickly pass through a heavy load area to complete the knife feeding action, thereby improving the firing capacity of the knife pushing member.

Referring to the description and the accompanying drawings below, specific embodiments of the present invention are disclosed in detail, and the manners in which the principles of the present invention may be adopted are indicated. It should be understood that the embodiments of the present invention are not limited in scope.

The features described and/or shown for one embodiment may be used in the same or similar manner in one or more other embodiments, and are combined with or substituted for features in other embodiments.

It should be emphasized that the term "include/comprise" when used herein refers to the presence of features, wholes, steps, or components, but does not exclude the presence or addition of one or more other features, wholes, steps, or components.

### Brief Description of the Drawings

To more clearly describe the technical solutions in the embodiments of the present invention or in the related art, the accompanying drawings that need to be used in the description of the embodiments or the related art will be briefly described below. Apparently, the accompanying drawings in the description below merely illustrate some embodiments of the present invention. Those of ordinary skill in the art may also derive other accompanying drawings from these accompanying drawings without creative efforts.
Fig. 1 is a schematic diagram of an external structure of an electric stapler according to an embodiment of the present invention;
Fig. 2 is a schematic diagram of an internal structure in a first view of an electric stapler according to an embodiment of the present invention;
Fig. 3 is a schematic diagram of an internal structure in a second view of an electric stapler according to an embodiment of the present invention;
Fig. 4 is a schematic diagram of a control circuit of an electric stapler according to an embodiment of the present invention;
Fig. 5 is a schematic diagram of a control circuit of an electric stapler according to another embodiment of the present invention;
Fig. 6 is a schematic diagram of a control circuit of an electric stapler according to another embodiment of the present invention;
Fig. 7 is a schematic flowchart of a control method for an electric stapler according to an embodiment of the present invention; and
Fig. 8 is a partial enlarged view of the electric stapler in Fig. 3.

### Description of reference signs:

100. electric stapler; 10. operating handle; 20. shaft assembly; 21. mandrel; 22. sleeve; 30. end effector; 31. staple cartridge seat; 32. staple abutting seat; 40. cutting knife assembly; 41. cutting knife; 42. knife pushing member; 60. trigger; 61. firing unit; 62. knife returning unit; 63. jaw closing unit; 64. jaw opening unit; 61'. jaw closing/firing unit; 62'. jaw opening/knife returning unit; 65. third position detection unit; 66. fourth position detection unit; 67. second position detection unit; 68. first position detection unit; 69. safety switch; 71. first button; 72. second button; 80. motor; 90. control module; 90', circuit board assembly; and 110. timing display module

### Detailed Description of the Embodiments

The technical solutions of the present invention will be described in detail in combination with the accompanying drawings and the specific embodiments below. It should be understood that these embodiments are only used to illustrate the present invention and not to limit the scope. Modifications made by those skilled in the art upon reading the present invention to various equivalent forms of the present invention fall all within the scope defined by the present invention.

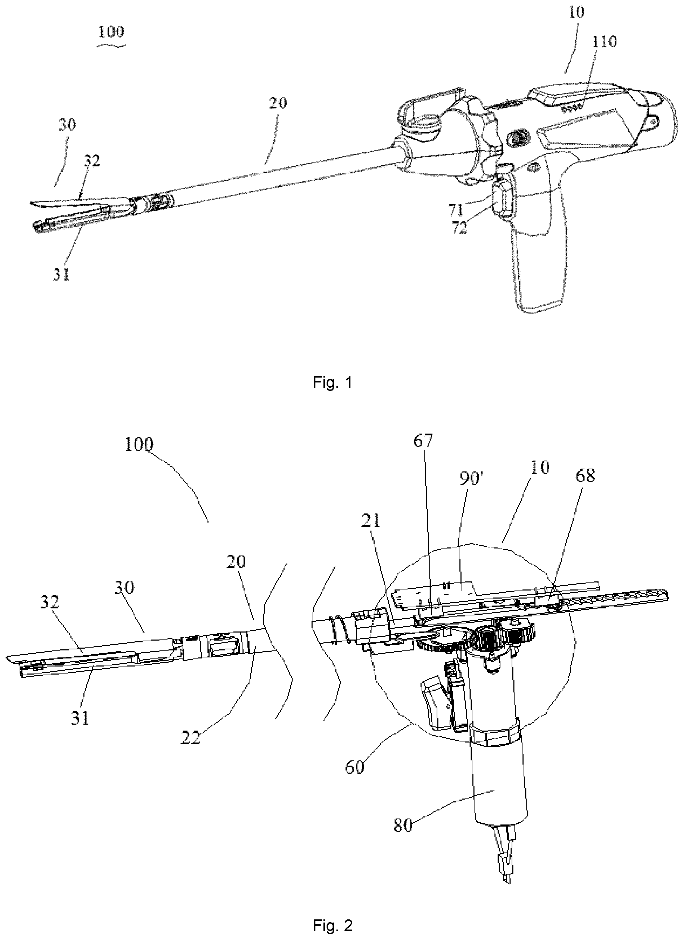

It should be noted that when an element is referred to as being "disposed" on another element, it may be directly located on another element or there may be a centered element. When one element is regarded to be "connected to" another element, it may be directly connected to another element or there may be a centered element simultaneously. An electric stapler and a control method therefor according to an embodiment of the present invention will be explained and illustrated in combination with Fig. 1 to Fig. 7 below. It should be noted that for ease of illustration, in the embodiments of the present invention, the same reference signs represent the same parts. For brevity, in different embodiments, detailed descriptions of the same parts are omitted, and descriptions of the same parts may be cross-referenced and referenced. The "Connection" in the present invention includes electrical connection and mechanical connection.

Specifically, with reference to an orientation of an electric stapler shown in Fig. 1, the terms "front" and "rear" used herein are relative to a user who operates the electric stapler. The term "rear" refers to a direction that is relatively close to the user, while the term "front" refers to a direction that is relatively away from the user. With respect to definitions of "left" and "right" directions, for example, an end effector is located on the "left", and a sleeve is located on the "right". The terms "up" and "down" refer to relative positions of a staple abutting seat and a staple cartridge seat of the end effector. Specifically, the staple abutting seat is located in a direction of "up" and the staple cartridge seat is located in a direction of "down". It should be understood that the orientations "up", "down", "front", "rear", "left", and "right" are defined for ease of description. However, the electric stapler may be used in many directions and positions, so that these terms used to express relative positional relationships are not limited or absolute. The embodiments described below with reference to the accompanying drawings are exemplary and intended to explain the present invention, and should not be construed as a limitation to the embodiments of the present invention.

Referring to Fig. 1 to Fig. 6, this embodiment provides an electric stapler 100, including an operating handle 10, a shaft assembly 20 extending from the operating handle 10, and an end effector 30 provided at one end of the shaft assembly 20. The operating handle 10 internally includes a power module and a motor 80. The power module is able to include a plurality of batteries connected in series, and provides electric energy for operation of the motor 80. The shaft assembly 20 includes a mandrel 21 and a sleeve 22 sleeved on the mandrel 21.

The electric stapler 100 further includes a transmission mechanism connected to the motor 80. When the motor 80 obtains the electric energy to operate, the motor 80 outputs power to drive the transmission mechanism to move. The mandrel 21 of the shaft assembly 20 has two ends, including one end connected to the transmission mechanism and the other end located in the sleeve 22. The sleeve 22 has one end connected to the transmission mechanism and the other end connected to the end effector 30.

The end effector 30 includes a jaw assembly and a staple cartridge assembly. The jaw assembly includes a staple cartridge seat 31 and a staple abutting seat 32 pivotally connected to the staple cartridge seat 31. The staple cartridge assembly is accommodated in the staple cartridge seat 31, and the staple abutting seat 32 is opened and closed relative to the staple cartridge seat 31, so as to clamp or release a tissue in cooperation with the staple cartridge seat 31 and the staple cartridge assembly. The sleeve 22 of the shaft assembly 20 has one end connected to the staple abutting seat 32 and the other end connected to the transmission mechanism. The transmission mechanism is driven by the motor 80 to drive the sleeve 22 to move in a front-rear direction. For example, the sleeve 22 is driven to move backward so that the staple abutting seat 32 pivots upward to open the jaw assembly, and the sleeve 22 is driven to move forward so that the staple abutting seat 32 pivots downward to close the jaw assembly.

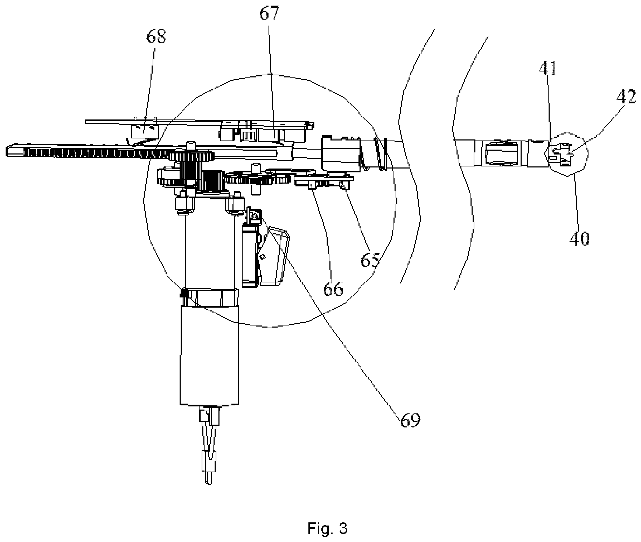

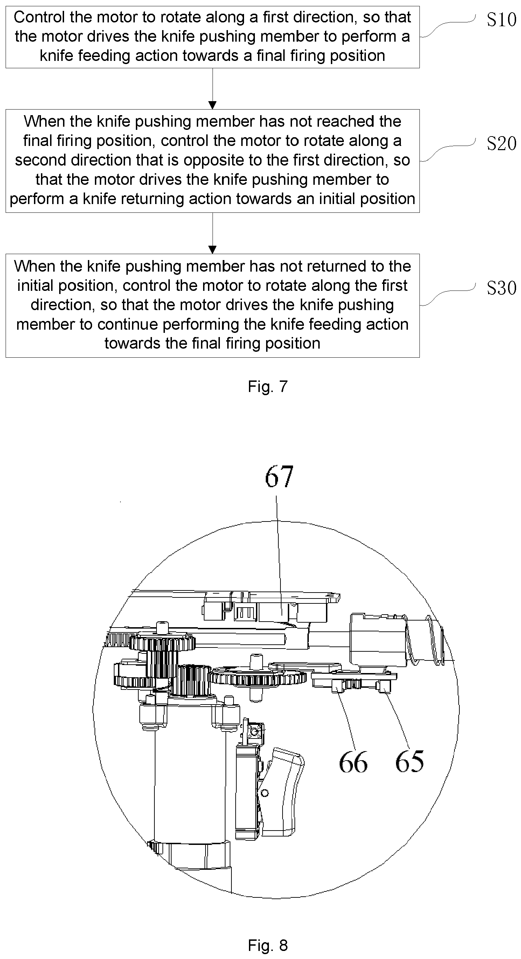

As shown in Fig. 3 and Fig. 8, the electric stapler further includes a cutting knife assembly 40 provided at one end, away from the operating handle 10, of the shaft assembly 20, where the cutting knife assembly 40 includes a knife pushing member 42 and a cutting knife 41 located at a front end of the knife pushing member 42. The knife pushing member 42 has a long sheet-shaped structure, and a part thereof is located in the sleeve 22 and connected to the mandrel 21. The transmission mechanism is able to drive the knife pushing member 42 to move forward and backward in the process of driving the mandrel 21 to move forward and backward. During the cutting and stapling, another part of the knife pushing member 42 is in contact with the cutting knife 41. During the forward movement of the knife pushing member 42, the cutting knife 41 is driven to move forward, so that the tissue is cut and a staple of the staple cartridge assembly is pushed to be discharged to staple the tissue.

There are two manners for connecting the knife pushing member 42 and the cutting knife 41. The first manner is to fixedly connect the knife pushing member 42 to the cutting knife 41. During the cutting and stapling, the cutting knife 41 is located in a space formed between the staple cartridge seat 31 and the staple abutting seat 32 of the jaw assembly. Due to being fixedly connected to the knife pushing member 42, the cutting knife is driven by the knife pushing member 42 to move forward and backward. The second manner is to separably connect the knife pushing member 42 to the cutting knife 41. In this manner, the cutting knife 41 is mounted in the staple cartridge assembly and is located in the staple cartridge seat 31 with the mounting of the staple cartridge assembly. During the cutting and stapling, the knife pushing member 42 is driven by the mandrel 21 to move forward until abutting against the cutting knife 41. When the knife pushing member 42 continues being driven by the mandrel 21 to move forward, the cutting knife 41 is able to be driven to move forward. When the knife pushing member 42 is driven by the mandrel 21 to move backward, the knife pushing member 42 separates from the cutting knife 41, so that the cutting knife 41 is left in an original position, cannot move backward together with the knife pushing member 42, and is taken out when the staple cartridge assembly is replaced subsequently.

In the first manner for connecting the knife pushing member 42 to the cutting knife 41, the cutting knife 41 is fixedly connected to the knife pushing member 42. The cutting knife 41 is reusable, without being replaced when the staple cartridge assembly is mounted each time, so that the user experience is more convenient. In the second manner for connecting the knife pushing member 42 to the cutting knife 41, when the knife pushing member 42 performs the knife returning action, the cutting knife 41 separates from the knife pushing member 42, so that even if the knife pushing member 42 fails to return to an initial position, the replacement of the staple cartridge assembly is not affected, and the higher safety is achieved.

In view of this, firstly, the motor 80 drives the jaw assembly by the transmission mechanism to be closed to clamp the tissue; then, the motor 80 drives the knife pushing member 42 and pushes the cutting knife 41 by the transmission mechanism to move forward to cut and staple the tissue, and the motor 80 drives the knife pushing member 42 by the transmission mechanism to move backward; and finally, the motor 80 drives the jaw assembly by the transmission mechanism to be opened to release the tissue, thus achieving the overall function of cutting and stapling of the stapler.

As shown in Fig. 2, the electric stapler 100 includes a trigger 60 and a control module 90, and is connected to a power module. The control module 90 is accommodated in the operating handle 10, and is specifically a circuit board assembly 90'. The control module 90 is electrically connected to the trigger 60 and the motor 80, and includes a detection unit, a control unit, a switch drive unit, and a switch unit. The detection unit detects a state of the trigger 60 and transmits a corresponding signal to the control unit when triggered. The control unit analyzes and processes the signal, and then performs control on the stapler. The control unit is able to be in the form of, for example, a microprocessor or processor, a computer-readable medium storing computer-readable program codes (such as software or firmware) that is able to be executed by the (micro) processor, a logic gate, a switch, an application specific integrated circuit (ASIC), a programmable logic controller, and an embedded microcontroller.

The trigger 60 includes a motor control trigger for controlling an operating state of the motor. When receiving a signal for controlling the motor 80 from this type of trigger, the control unit generates a corresponding low-voltage pulse width modulation (PWM) signal to the switch drive unit, and the switch drive unit converts the input low-voltage PWM signal into a high-voltage PWM signal usable for the switch unit and outputs the high-voltage PWM signal to the switch unit, so as to control switch-on/off of the switch unit. The power module is connected to two ends of the motor 80 through the switch unit, and a switch-on/off frequency of the switch unit determines whether the power module supplies power to or disconnects power from the motor 80, so as to determine the operating state of the motor 80, namely, a rotational speed and direction of the motor.

In this embodiment, the switch unit includes four Mos switches that constitute an H-bridge, switch-on/off of the Mos switches of the H-bridge determines whether the power module supplies power to or disconnects power from the motor 80, and the switch drive unit is a Mos drive chip. Moreover, due to design of the H-bridge, the control unit controls the H-bridge to implement forward or reverse rotation of the motor. It should be noted that the above H-bridge control and forward and reverse rotation control of the motor are both existing technologies and will not be repeated. The control unit is a microcontroller chip.

To implement a tissue cutting function of the cutting knife assembly 40, the motor control trigger includes: a firing unit 61 for a user to trigger and a knife returning unit 62 for the user to trigger, where the firing unit 61 is configured to send a firing signal representing a knife feeding action and a firing stopping signal representing stopping of the knife feeding action, and the knife returning unit 62 is configured to send a knife returning signal representing a knife returning action and a knife returning stopping signal representing stopping of the knife returning action. Specifically, the user is able to input a signal by touching, pressing or releasing the firing unit 61 and the knife returning unit 62, to control start and stop of the motor 80.

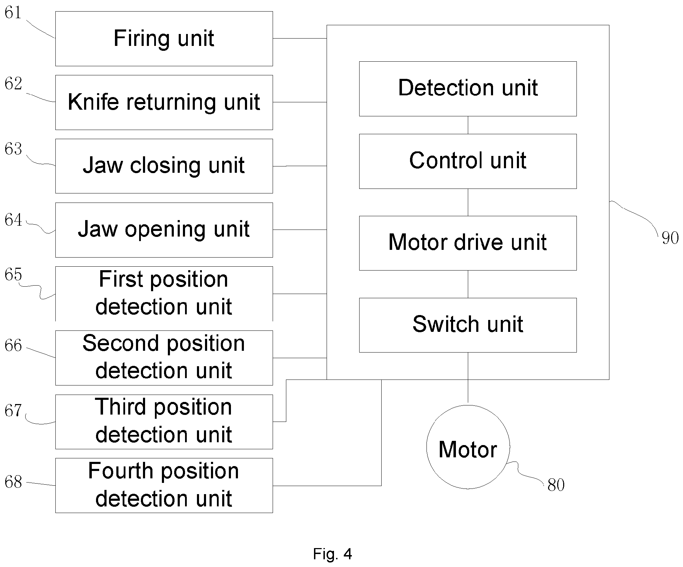

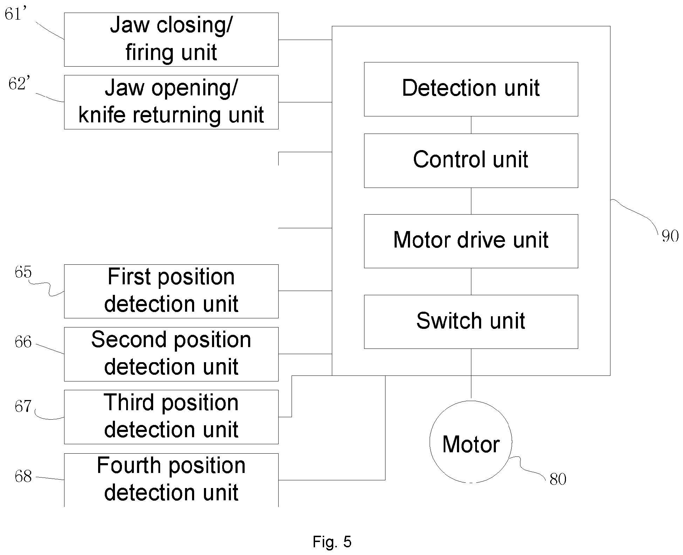

In one embodiment, as shown in Fig. 4 and Fig. 5, the firing unit 61 and the knife returning unit 62 have one button disposed on the operating handle 10, respectively, which is able to be triggered by the user when operating the handle 10. The button corresponding to the firing unit 61 is a first button 71, and the button corresponding to the knife returning unit 62 is a second button 72. The user is able to trigger a corresponding signal by pressing the button or releasing the button after pressing, and inputs an electrical signal to the control unit through the detection unit of the control module 90.

Both the firing unit 61 and the knife returning unit 62 may be normally open switches. The firing unit 61 and the knife returning unit 62 are able to output corresponding signals according to the trigger of the user. The control module 90 receives the signals from the firing unit 61 and the knife returning unit 62, respectively, to control the operating state, namely, the start or stop of the motor, so as to perform corresponding actions of the stapler.

In this embodiment, the user operates the first button 71 to trigger the firing unit 61, and the control module 90 controls, when detecting the firing signal, the motor 80 to operate for driving the knife pushing member 42 to perform the knife feeding action towards a final firing position. When the first button 71 stops being operated, the firing unit 61 is not triggered, and the control module 90 controls, when detecting the firing stopping signal, the motor 80 to stop.

During use, different corresponding signals are given according to trigger states of the firing unit 61. The trigger states include triggering and non triggering. When the user triggers the firing unit 61, a high-level signal is output, and when the user does not trigger the firing unit 61, a low-level signal is output. The firing signal is a sudden change from a low level to a high level, and the firing stopping signal is a sudden change from the high level to the low level, so that when the user continuously triggers the firing unit 61, the knife pushing member 42 continuously performs a firing action, and when the firing unit 61 stops being triggered, the firing action immediately stops.

Alternatively, the firing signal is an odd-numbered sudden change from the low level to the high level, and the firing stopping signal is an even-numbered sudden change from the low level to the high level. Consequently, when the user operates the first button 71 for the first time, the firing unit 61 is triggered, and the control module 90 controls, when detecting the firing signal, the motor 80 to operate; and when the user operates the first button 71 again, the firing unit 61 is fired again, and the control module 90 controls, when detecting the firing stopping signal, the motor 80 to stop. Alternatively, in some embodiments, the firing unit 61 further includes a first timing module in which a preset time such as 0.5 s is set. When the firing unit 61 is triggered, the first timing module is switched on simultaneously and starts timing. When a counted time reaches the preset time of 0.5 s, the first timing module is switched off and the counted time is reset. The control module 90 determines, when detecting that the counted time is reset, the signal as the firing stopping signal and controls the motor 80 to stop. Thus, the control module 90 is configured to determine, when detecting that the counted time of the first timing module of the firing unit 61 is reset, the signal as the firing stopping signal and control the motor 80 to stop, and to determine, when detecting that the first timing module is switched on, the signal as the firing signal and control the motor 80 to operate.

In this embodiment, during knife feeding, when the firing stopping signal is detected by the control module 90, the motor 80 is controlled to stop and the knife pushing member 42 stops moving. If the knife pushing member 42 stops, during the knife feeding action, at any intermediate point between the initial position and the final firing position, the user triggers the knife returning unit 62 by operating the second button 72, and the control module 90 controls, when detecting the knife returning signal, the motor 80 to operate, so that the knife pushing member 42 is driven to perform the knife returning action, during the knife returning action, the control module 90 controls, when detecting the knife returning stopping signal, the motor 80 to stop, and the knife pushing member 42 stops the knife returning action.

In this embodiment, gradual fire is able to be implemented by the firing unit 61. When the knife feeding action stops, gradual knife returning is implemented by the knife returning unit 62. Unlike a conventional electric stapler 100 in which the knife pushing member 42 directly returns to the initial position during knife returning, the knife returning unit 62 is able to perform a knife returning action of not directly returning to the initial position by the knife pushing member 42, so that more observation and operation time is provided, and a doctor is able to perform independent control according to complex surgical conditions, thereby enhancing the operability, better serving a surgical process, and achieving good user experience. For example, the gradual knife returning process provides more operation time, so that the doctor is able to accurately put the cut tissue into a disposable medical bag, thereby avoiding contamination of healthy tissues; and finally the bag is taken.

During use, the user is able to operate the second button 72 to give a corresponding signal according to a trigger state of the knife returning unit 62. A principle of outputting the knife returning signal and the knife returning stopping signal by the knife returning unit 62 is the same as that of the above firing unit 61, and will not be repeated herein in the present invention.

The knife pushing member 42 is driven by the transmission mechanism to move, and according to a working mode of the stapler used in a surgery, the knife pushing member 42 has a movement stroke of a fixed length and is driven by the transmission mechanism to move forward or backward within the movement stroke. A farthest end of the movement stroke is a front stop point of the forward movement, that is the aforementioned final firing position. A nearest end of the movement stroke is a starting point of the forward movement or a rear stop point of the backward movement, which is the aforementioned initial position. Before being fired, the knife pushing member 42 is located in the initial position; when being fired, the knife pushing member 42 moves from the initial position to the final firing position to cut the tissue, and stops at the intermediate point of the movement stroke, which is a position between the initial position and the final firing position, or finally reaches the final firing position; and during knife returning, the knife pushing member 42 moves backward from the intermediate point or the final firing position.

In a case where the knife pushing member 42 is not fully fired, according to the knife feeding action and the knife returning action of the knife pushing member 42, it is defined that the knife pushing member 42 has, during the knife feeding action, a first state of stopping between the initial position and the final firing position, that is, the knife pushing member 42 stops at the intermediate point of the movement stroke during knife feeding, and the knife pushing member has a second state of performing the knife returning action in the first state and stopping between the initial position and the final firing position, that is, the knife pushing member stops at the intermediate point of the movement stroke when performing the knife returning action in the first state. When the user sends the firing stopping signal by the firing unit 61, the knife pushing member 42 stops at a first intermediate point of the movement stroke, that is, the knife pushing member is not fully fired and is in the first state, and then, by the knife returning unit 62, the knife pushing member 42 stops at a second intermediate point of the movement stroke after knife returning, that is, the knife pushing member has not returned to the initial position and is in the second state.

In both the first state and the second state, the firing unit 61 is in an effective state, and the control module 90 controls, when detecting the firing signal, the motor 80 to operate for driving the knife pushing member 42 to continue performing the knife feeding action. Consequently, in the first state, the knife pushing member 42 is able to perform the knife returning action of not directly returning to the initial position in a state that the knife pushing member has been fired but is not fully fired and stops moving forward, and is able to enter the second state after the knife returning action of not directly returning to the initial position in one step stops; and in the second state, the firing unit 61 is effective and is able to control the knife pushing member 42 to continue performing the knife feeding action, thereby implementing a "zipper" function.

Further, when the knife pushing member 42 is in the first state, a first stop position is provided at an end, close to the cutting knife 41, of the knife pushing member 42, which is the aforementioned first intermediate point; and when the knife pushing member 42 is in the second state, a second stop position is provided at an end, close to the cutting knife 41, of the knife pushing member 42, which is the aforementioned second intermediate point, and the second stop position is located behind the first stop position or coincides with the first stop position.

During knife feeding movement, when encountering a heavy cutting load, the cutting knife 41 at the front end is blocked and slowly moves forward or cannot move forward. In this case, due to its long sheet-shaped structure, the knife pushing member 42 is continuously pushed forward by the transmission mechanism at a rear end, so that the knife pushing member is prone to bending on an inner wall of the sleeve 22, and a larger frictional force is generated between the knife pushing member and the inner wall of the sleeve 22 to further prevent the knife feeding action. With the adoption of the above solution, in the first state, when the knife returning unit 62 is triggered to control performing of the knife returning action, the knife pushing member 42 is able to recover to be in a straight state. In the process that the knife pushing member 42 recovers to be in the straight state, the rear end, which is connected to the mandrel 21 and is away from the cutting knife 41, of the knife pushing member 42 is mainly pulled back by the transmission mechanism to recover the knife pushing member 42 to be in from a bent state to the straight state, and the front end, close to the cutting knife 41, of the knife pushing member 42 is able to remain unchanged or move backward for a distance.

When the knife pushing member 42 reaches the final firing position, the knife pushing member 42 has been fully fired, the control module 90 controls the firing unit 61 to be in a failed state, and the knife pushing member 42 no longer performs the knife feeding action.

When the knife pushing member 42 is in the first state, the control module 90 controls, when detecting the knife returning signal, the knife pushing member 42 performs the knife returning action towards the initial position. During the knife returning action, when detecting the knife returning stopping signal, the knife pushing member 42 is controlled to stop the knife returning action. When the knife pushing member 42 stops the knife returning action and has not reached the initial position, that is, when the knife pushing member 42 has not completed the knife returning action and is not fully fired, the firing unit 61 is in the effective state. When the firing unit 61 is triggered, the knife pushing member 42 is able to still continue feeding the knife. Consequently, the "zipper" function of the knife pushing member 42 is able to improve the firing capacity of the electric stapler 100. For example, when encountering the heavy cutting load, the knife pushing member 42 is prone to bending on the inner wall of the sleeve 22 during the knife feeding movement, and the larger frictional force is generated between the knife pushing member 42 and the inner wall of the sleeve 22, making it impossible to continue performing the knife feeding action. If the knife pushing member 42 is controlled to perform the knife returning action for a distance by triggering the knife returning unit 62, the knife pushing member 42 is able to recover to be in the straight state, so that there is no friction between the knife pushing member 42 and the inner wall of the sleeve 22. In this case, by triggering the firing unit 61, the knife pushing member is able to quickly pass through a heavy load area to complete the knife feeding action.

Meanwhile, the "zipper" function of the knife pushing member 42 is able to improve the fault tolerance capability of the electric stapler 100. During normal use, when the knife pushing member 42 is gradually fired to stop at the intermediate point, if the user mistakenly triggers the knife returning unit 62 to cause the knife pushing member 42 to perform the knife returning action and stop, the user is able to operate the firing unit 61 again to implement re-fire as long as the knife pushing member is not returned to the initial position, so that the remaining staple cartridge is able to continue being used up and the ideal results is able to be achieved for the surgery.

In the first state, the control module 90 controls, when detecting the knife returning signal, the knife pushing member 42 to perform the knife returning action towards the initial position, and when the knife pushing member 42 stops the knife returning action and reaches the initial position, that is, when the knife pushing member 42 completes the knife returning action, the control module 90 controls the firing unit 61 to switch to be in from the effective state to the failed state, and the knife pushing member 42 cannot be fired again, to prevent re-cutting a stapling position to cause injury of a patient.

The failed state of the above firing unit 61 refers to that when the firing unit is triggered, the stapler does not perform the action. The effective state refers to that when the unit is triggered, the stapler performs the corresponding action. In this embodiment, when the firing unit 61 is triggered in the failed state, the firing unit 61 still sends a signal to the control module 90, but the control module 90 neither analyzes and processes the signal nor instructs, according to the signal, the motor 80 to act. On the contrary, when the firing unit 61 is triggered in the effective state, the control module 90 controls the motor 80 to rotate forward according to the signal.

In this embodiment, the motor control trigger further includes a first position detection unit 68 and a second position detection unit 67, to control stop of the motor 80. The first position detection unit 68 and the second position detection unit 67 are provided in the operating handle 10 and located in a position where the user cannot directly trigger them with a hand. The first position detection unit 68 and the second position detection unit 67 are configured to detect a position of the cutting knife assembly 40 to determine whether the knife pushing member 42 is fully fired or returns to the initial position, and to make the motor 80 stop by the control module 90 so as to make the knife pushing member 42 stop moving.

In this embodiment, the transmission mechanism includes a matching portion, where the matching portion includes a first bump and a second bump. The first position detection unit 68 and the second position detection unit 67 are able to be switch mechanisms. In the process that the transmission mechanism drives the knife pushing member 42 and the cutting knife 41 by the mandrel 21 to move forward, the front stop point, namely, the final firing position is reached, the first bump triggers the second position detection unit 67, the control module 90 receives a second in-place signal sent by the second position detection unit 67 and instructs the motor 80 to stop rotating, and the cutting knife assembly 40 is fully fired. In the process that the transmission mechanism drives the knife pushing member 42 by the mandrel 21 to move backward, the rear stop point, namely, the initial position is reached, the second bump triggers the first position detection unit 68, the control module 90 receives a first in-place signal sent by the first position detection unit 68 and instructs the motor 80 to stop rotating, and the knife pushing member 42 completes knife returning, that is, the knife pushing member returns to the initial position. It should be noted that the first bump and the second bump are able to be substituted with one bump, and the above one bump triggers the second position detection unit 67 during forward movement and the first position detection unit 68 during backward movement. In this case, the matching portion includes the above one bump.

Further, when the user triggers the firing unit 61 and keeps triggering, the motor 80 drives the knife pushing member 42 by the transmission mechanism to perform the knife feeding action until the cutting knife 41 at the front end of the knife pushing member 42 triggers the second position detection unit 67, and after receiving the second in-place signal, the control module 90 controls the motor 80 to stop and the firing unit 61 to be in the failed state, so as to control the cutting knife assembly 40 to no longer perform knife feeding.

In one embodiment, the control module 90 is able to control, based on the second in-place signal, the motor 80 to stop, and drive, when detecting the firing stopping signal, the motor 80 to control the knife pushing member 42 to perform the knife returning action until the initial position is reached. For example, during knife feeding, when the user triggers the firing unit 61 and keeps triggering, the knife pushing member 42 is able to continue performing the knife feeding action until the second position detection unit 67 is triggered, and the control unit controls the motor 80 to stop. In this case, when the user no longer triggers the firing unit 61 to give the firing stopping signal, the control unit controls the motor 80 to drive the knife pushing member 42 to perform the knife returning action, and the knife pushing member 42 moves backward and stops until the first position detection unit 68 is triggered. Thus, when the knife pushing member 42 is fully fired, the knife pushing member 42 is automatically returned in one step by releasing the firing unit 61, so that operation time of the user is saved.

In one embodiment, the control module 90 is able to control the motor 80 based on the second in-place signal to stop, and control the knife pushing member 42 based on the detected knife returning signal to perform the knife returning action. When the knife pushing member 42 is fully fired, the second position detection unit 67 is triggered by the matching portion of the transmission mechanism, and the control module 90 receives the second in-place signal to control the motor 80 to stop. When knife returning is required, the user operates the knife returning unit 62 to give the knife returning signal, and after detecting the knife returning signal, the control module 90 controls the motor 80 to drive the knife pushing member 42 to perform the knife returning action.

In this embodiment, when the knife pushing member 42 performs the knife feeding action and reaches the final firing position, the control module 90 controls, when detecting the knife returning signal, the motor 80 to drive the knife pushing member 42 to leave the final firing position and perform the knife returning action towards the initial position until the initial position is reached. Specifically, when the knife pushing member 42 performs the knife feeding action and reaches the final firing position, the second position detection unit 67 is triggered by the matching portion of the transmission mechanism, and the control module 90 controls, when receiving the second in-place signal, the motor 80 to stop. In this case, when the user triggers the knife returning unit 62, the control module 90 controls, after detecting the knife returning signal, the motor 80 to drive the knife pushing member 42 to perform the knife returning action and stop until the first position detection unit 68 is triggered, so that a manner of manually returning to the initial position is implemented regardless of whether the knife returning stopping signal is received halfway. In this manner, the knife returning unit 62 provides a start signal for the knife returning action of directly returning to the initial position. In the process of directly returning to the initial position, the user does not need to keep continuous trigger of the knife returning unit 62, and the firing unit 61 is always kept in the failed state. Alternatively, when the knife pushing member 42 performs the knife feeding action and reaches the final firing position, the control module 90 controls, when detecting the knife returning signal, the motor 80 to drive the knife pushing member 42 to perform the knife returning action towards the initial position. During the knife returning action, the control module 90 controls, when detecting the knife returning stopping signal, the knife pushing member 42 to stop the knife returning action.

The knife pushing member 42 has a third state of performing the knife returning action after reaching the final firing position and stopping between the initial position and the final firing position, and in the third state, the control module 90 controls the firing unit 61 to be kept in the failed state, so as to control the knife pushing member 42 to no longer perform knife feeding. When the knife pushing member 42 reaches the final firing position, the control module 90 controls, when detecting the knife returning signal, the motor 80 to drive the knife pushing member 42 to perform the knife returning action towards the initial position. During the knife returning action, the control module 90 controls, when detecting the knife returning stopping signal, the knife pushing member 42 to stop the knife returning action, that is, the knife pushing member is in the third state. Unlike the first state and the second state of the knife pushing member 42, the firing unit 61 is kept in the failed state when the knife pushing member 42 is in the third state, so that the knife pushing member 42 cannot perform the knife feeding action anymore.

In some embodiments, when the user triggers the knife returning unit 62, the control module 90 controls, when detecting the knife returning signal, the motor 80 to operate for driving the knife pushing member 42 of the cutting knife assembly 40 to perform the knife returning action towards the initial position; and when the user does not trigger the knife returning unit 62, the control module 90 controls, when detecting the knife returning stopping signal, the motor 80 to stop. Consequently, during use, when the user keeps continuous trigger of the knife returning unit 62, the knife pushing member 42 is able to continuously perform the knife returning action and automatically stop until the first position detection unit 68 is triggered. Alternatively, when the user triggers the knife returning unit 62, the knife pushing member 42 performs the knife returning action. During the knife returning, once the user does not trigger the knife returning unit 62, the motor 80 immediately stops, and the knife pushing member 42 stops the knife returning action, so that the knife pushing member 42 performs the knife returning action in stages (performs the knife returning gradually) by triggering the knife returning unit 62 for multiple times and automatically stops until the first position detection unit 68 is triggered, and thus a manner of manually the knife returning in stages until the knife pushing member 42 is returned to the initial position is implemented.

The knife feeding action and the knife returning action of the knife pushing member 42 is able to be implemented by forward and reverse rotation of the motor 80. That is, a rotation direction of an output shaft of the motor 80 includes a first direction and a second direction opposite to each other. If the first direction is defined as a forward rotation direction, the second direction is a reverse rotation direction. When the control unit drives the motor 80 to rotate forward, the motor 80 is able to drive the knife pushing member 42 by the transmission mechanism to perform the knife feeding action. When the control unit drives the motor 80 to rotate reversely, the motor 80 is able to drive the knife pushing member 42 by the transmission mechanism to perform the knife returning action.

In the present invention, the same motor 80 is used to provide power for the knife feeding action and the knife returning action of the knife pushing member 42 and a jaw opening action and a jaw closing action of the jaw assembly. Within one execution cycle, the electric stapler needs to sequentially complete the jaw closing action, the firing action, the knife returning action, and the jaw opening action to complete an entire working process. The above four actions corresponding to the entire working process is able to be sequentially driven by sequentially driving forward rotation, forward rotation, reverse rotation, and reverse rotation of the motor 80 by the control module 90 to eventually implement sequential execution of the jaw closing action, the firing action, the knife returning action, and the jaw opening action.

To implement a jaw opening and closing function, the motor control trigger further includes: a jaw closing unit 63 for the user to trigger a jaw closing signal representing the jaw closing action; and a jaw opening unit 64 for the user to trigger a jaw opening signal representing the jaw opening action; and the control module 90 controls, when detecting the jaw closing signal, the jaw assembly to perform the jaw closing action, that is, the staple abutting seat 32 pivots downward to close the jaw assembly, and the control module 90 controls, when detecting the jaw opening signal, the jaw assembly to perform the jaw opening action, that is, the staple abutting seat 32 pivots upward to open the jaw assembly. Specifically, the user is able to input a signal by touching or pressing the jaw closing unit 63 and the jaw opening unit 64, to control start and stop of the motor 80.

In one embodiment, as shown in Fig. 4, the jaw opening unit 64 and the jaw closing unit 63 have one button disposed on the operating handle 10, respectively. The button corresponding to the jaw closing unit 63 is a third button, and the button corresponding to the jaw opening unit 64 is a fourth button. The user is able to trigger a corresponding signal by pressing the button, and inputs an electrical signal to the control unit through the detection unit of the control module 90. The user triggers the jaw closing unit 63 by operating the third button, the control module 90 controls the motor 80 to rotate forward to drive the jaw assembly to perform the jaw closing action, and when the third button stops being operated, the jaw closing unit 63 is not triggered, and the control module 90 controls the motor 80 to stop; and the user triggers the jaw opening unit 64 by operating the fourth button, the control module 90 controls the motor 80 to rotate reversely to drive the jaw assembly to perform the jaw opening action, and when the fourth button stops being operated, the jaw opening unit 64 is not triggered, and the control module 90 controls the motor 80 to stop.

In another embodiment, as shown in Fig. 5, the jaw closing unit 63 and the firing unit 61 are integrated as a jaw closing/firing unit 61', which is specifically the first button 71, and the knife returning unit 62 and the jaw opening unit 64 are integrated as a jaw opening/knife returning unit 62', which is specifically the second button 72, so that a firing switch for controlling forward rotation of the motor 80 is a same switch unit. The stapler completes the entire working process by sequentially performing the jaw closing action, the firing action, the knife returning action, and the jaw opening action. The above four actions corresponding to the entire working process are sequentially completed by sequentially pressing the first button 71, the first button 71, the second button 72, and the second button 72 to drive forward rotation, forward rotation, reverse rotation, and reverse rotation of the motor 80 by the control module 90, finally sequential completion of all actions described above.

Further, during pivoting movement, the staple abutting seat 32 bidirectionally moves between a final closing position and a final opening position, where the final closing position is a position where the staple abutting seat 32 pivots to be closest to the staple cartridge seat 31, and in this case, the jaw assembly is closed in place; and the final opening position is a position where the staple abutting seat 32 pivots to be farthest from the staple cartridge seat 31, and in this case, the jaw assembly is opened in place. The motor control trigger further includes a third position detection unit 65 and a fourth position detection unit 66, to control stop of the motor 80. The third position detection unit 65 is configured to detect whether the jaw assembly is closed in place and to make the motor 80 stop by the control module 90 so as to make the staple abutting seat 32 stop pivoting. The fourth position detection unit 66 is configured to detect whether the jaw assembly is opened in place and to make the motor 80 stop by the control module 90 so as to make the staple abutting seat 32 stop pivoting.

In this embodiment, the matching portion of the transmission mechanism further includes a third bump and a fourth bump. The third position detection unit 65 and the fourth position detection unit 66 are able to be switch mechanisms. In the process that the transmission mechanism drives the staple abutting seat 32 by driving forward movement of the sleeve 22 to be closed and rotated, when the staple abutting seat 32 reaches the final closing position, the third bump triggers the third position detection unit 65, the control module 90 receives a signal sent by the third position detection unit 65 and instructs the motor to stop forward rotation, and the jaw assembly has been closed in place and is in a jaw closing state. In the process that the transmission mechanism drives the staple abutting seat 32 by driving backward movement of the sleeve 22 to be opened and rotated, when the staple abutting seat 32 reaches the final opening position, the fourth bump triggers the fourth position detection unit 66, the control module 90 receives a signal sent by the fourth position detection unit 66 and instructs the motor to stop backward rotation, and the jaw assembly has been opened in place and is in a final jaw opening state. It should be noted that the third bump and the fourth bump are able to be substituted with one bump.

The transmission mechanism is able to drive the sleeve 22 by a first assembly to move to drive the jaw assembly to be opened and closed. The transmission mechanism drives the mandrel 21 by a second assembly to move to drive the knife pushing member 42 to feed and the knife returning action. The first assembly and the second assembly do not act simultaneously, to meet an action logic relationship between the jaw assembly and the knife pushing member 42. The first assembly includes a first bump and a second bump, or the first assembly includes one bump substituted for the first bump and the second bump. The second assembly includes a third bump and a fourth bump, or the second assembly includes one bump substituted for the third bump and the fourth bump.

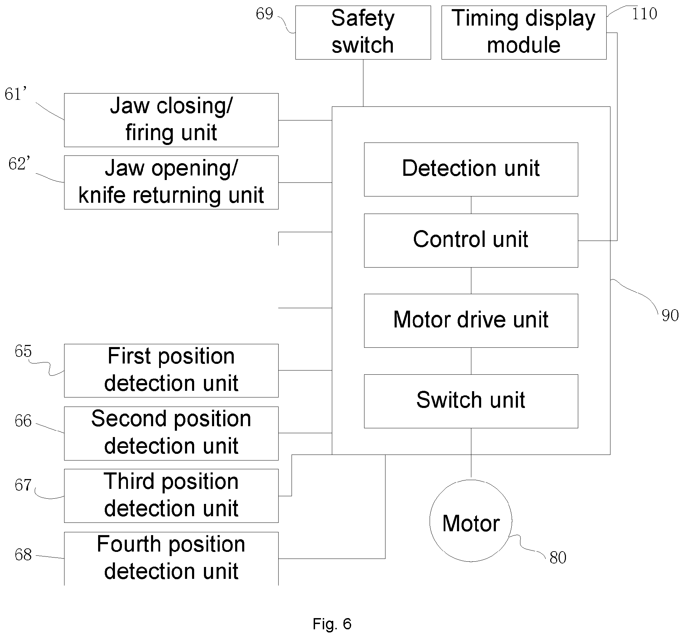

In another embodiment, as shown in Fig. 6, the trigger 60 further includes a safety switch 69 manually triggered by user operation and electrically connected to the control module 90. The safety switch 69 has one button disposed on the operating handle 10. The user is able to operate the button to trigger the corresponding switch. The switch inputs an electrical signal to the control unit through the detection unit. The control module 90 further includes a timing unit in which a preset time such as 15 s is set. The timing unit is switched on and starts timing, and when a counted time reaches the preset time, the timing unit is switched off and the counted time is reset. The timing unit is able to be a circuit module independent of a microcontroller or a timing unit of the microcontroller. The stapler further includes a timing display module 110 electrically connected to the control unit and having two functions: a first function for indicating that the timing unit reaches the preset time and informs the user of the state; and a second function for indicating a real-time time length of timing work of the timing unit and providing the real-time time length for the user to obtain an elapsed time length. When the timing unit is switched on, the control module 90 simultaneously switches on the timing display module 110, for example, if a time counted by the timing unit is 10 s, the timing display module 110 indicates that an elapsed time is 10 s. Within one operating cycle, the safety switch 69 is able to only be effectively triggered when the jaw assembly switches to be in from the opening state to the closing state, and both the timing unit and the timing display module 110 are switched on. During timing, the user cannot operate the failed firing unit 61 to perform the firing action. Only when the timing unit is switched off, the firing unit 61 becomes effective, so that the subsequent firing action is able to be performed.

In view of this, the safety switch 69, the timing unit, and the timing display module 110 form a pressing and keeping mechanism that facilitates the user to know the tissue that has been fully pressed. The pressing and keeping mechanism is able to cause the jaw assembly to press the target tissue for a certain period of time, so that a tissue fluid is eliminated more fully, that is, the pressing and keeping mechanism is able to prevent the knife pushing member 42 from moving forward within a certain period of time and keep the jaw assembly closed to improve the pressing effect of the jaw assembly on the tissue.

In other embodiments, dual motors are used to provide a driving force. One motor drives the knife pushing member 42 to perform the firing action and the knife returning action, while the other motor drives the jaw assembly to perform the jaw opening action and the jaw closing action. In this embodiment, the above first to fourth position detection units are able to also be used, a difference is that the controlled motors are different, the above knife returning unit and a control logic of the knife returning unit are able to also be used, and the same "zipper" function, the above gradual knife returning function and so on are implemented, which are easily understood by those skilled in the art and will not be repeated herein.

The present invention further provides a control method for an electric stapler. As shown in Fig. 1 to Fig. 7, the electric stapler 100 includes a cutting knife assembly 40 and a motor 80, where the cutting knife assembly 40 includes a knife pushing member 42, and the motor 80 is configured to drive the knife pushing member 42 to move. The control method includes:
Step S10: controlling the motor 80 to rotate along a first direction, so that the motor 80 drives the knife pushing member 42 to perform a knife feeding action towards a final firing position;
Step S20: when the knife pushing member 42 has not reached the final firing position, controlling the motor 80 to rotate along a second direction that is opposite to the first direction, so that the motor 80 drives the knife pushing member 42 to perform a knife returning action towards an initial position; and
Step S30: when the knife pushing member 42 has not returned to the initial position, controlling the motor 80 to rotate along the first direction, so that the motor 80 drives the knife pushing member 42 to continue performing the knife feeding action towards the final firing position.

In one embodiment, if the first direction is a forward rotation direction, the second direction is a reverse rotation direction. When the control unit drives the motor 80 to rotate forward, the motor 80 is able to drive the knife pushing member 42 to perform the knife feeding action. When the control unit drives the motor 80 to rotate reversely, the motor 80 is able to drive the knife pushing member 42 to perform the knife returning action.

According to the control method, when the knife pushing member 42 is not fully fired and the user mistakenly triggers the knife pushing member 42 to return, the motor 80 is controlled to stop, so that the knife returning action stops. When the knife pushing member 42 has not reached a rear stop point, the motor 80 is controlled to rotate forward, to drive the knife pushing member 42 to be fired again, so that the remaining staple cartridge in the jaw assembly is able to continue being used and the ideal results is able to be achieved for a surgery.

In a case where the knife pushing member cannot be fired when encountering a heavy load, the motor 80 is controlled to rotate reversely, so that the knife pushing member 42 is able to recover to be in a straight state after being driven by the motor 80 to perform the knife returning action for a distance. Then the motor 80 is controlled to rotate forward, so that the motor 80 drives the knife pushing member 42 to quickly pass through a heavy load area, to complete the knife feeding action, thereby improving the firing capacity of the electric stapler 100.

The above embodiments are only intended to illustrate the technical concept and characteristics of the present invention to enable those skilled in the art to understand the content of the present invention and implement it accordingly, and should not limit the scope of protection of the present invention. Any equivalent changes or modifications made in accordance with the spirit and essence of the present invention should be covered by the scope of protection of the present invention.

All articles and references disclosed, including patent applications and publications, are incorporated herein by reference for various purposes. The term "substantially include/comprise" for describing a combination should include the determined elements, components, parts, or steps and other elements, components, parts, or steps that do not substantially affect basic novel features of the combination. The use of the term "comprise" or "include" to describe a combination of elements, components, parts, or steps herein also contemplates the embodiment that substantially includes these elements, components, parts, or steps. The use of the term "may" herein is intended to illustrate that any of the described attributes included in "may" are optional.

A plurality of elements, components, parts, or steps can be provided by a single integrated element, component, part, or step. Alternatively, a single integrated element, component, part, or step may be divided into a plurality of separated elements, components, parts, or steps. The disclosure of "a/an" or "one" used to describe an element, a component, a part, or a step is not intended to exclude other elements, components, parts, or steps.

It should be understood that the above description is intended to be illustrative and not restrictive. Many embodiments and many applications other than the examples provided will be apparent to those skilled in the art upon reading the above description. The disclosures of all articles and references, including patent applications and publications, are incorporated herein by reference for all purposes.

## Claims

1. An electric stapler, comprising:
an operating handle;
a shaft assembly, extending from the operating handle;
a cutting knife assembly, provided at one end, away from the operating handle, of the shaft assembly and comprising a knife pushing member;
a motor, provided in the operating handle and configured to drive the knife pushing member to move;
a trigger, comprising: a firing unit for a user to trigger, the firing unit being configured to send a firing signal representing a knife feeding action and a firing stopping signal representing stopping of the knife feeding action; and a knife returning unit for the user to trigger, the knife returning unit being configured to send a knife returning signal representing a knife returning action and a knife returning stopping signal representing stopping of the knife returning action; and
a control module, electrically connected to the motor and the trigger, where the control module controls, when detecting the firing signal, the motor to drive the knife pushing member to perform the knife feeding action towards a final firing position, and the control module controls, when detecting the firing stopping signal, the knife pushing member to stop the knife feeding action; and
the knife pushing member has, during the knife feeding action, a first state of stopping between an initial position and the final firing position, when in the first state, the control module controls, when detecting the knife returning signal, the knife pushing member to perform the knife returning action towards the initial position, and during the knife returning action, the control module controls, when detecting the knife returning stopping signal, the knife pushing member to stop the knife returning action.

2. The electric stapler as claimed in claim 1, wherein the knife pushing member has a second state of performing the knife returning action in the first state and stopping between the initial position and the final firing position, and in both the first state and the second state, the firing unit is in an effective state, so that the control module controls, based on the detected firing signal, the knife pushing member to perform the knife feeding action.

3. The electric stapler as claimed in claim 2, wherein the cutting knife assembly further comprises a cutting knife located in front of the knife pushing member towards the final firing position, and when the knife pushing member is in the first state, a first stop position is provided at an end, close to the cutting knife, of the knife pushing member; and when the knife pushing member is in the second state, a second stop position is provided at an end, close to the cutting knife, of the knife pushing member, and the second stop position is located behind the first stop position or coincides with the first stop position.

4. The electric stapler as claimed in claim 1, wherein when the knife pushing member is in the first state, the control module controls, when detecting the knife returning signal, the knife pushing member to perform the knife returning action towards the initial position, and when the knife pushing member stops the knife returning action and reaches the initial position, the control module controls the firing unit to switch to be in from an effective state to a failed state, so as to control the knife pushing member to no longer perform knife feeding.

5. The electric stapler as claimed in claim 1 or 4, wherein the trigger comprises a first position detection unit configured to detect whether the knife pushing member is located in the initial position and to output a first in-place signal representing that the knife pushing member is located in the initial position, and the control module controls, based on the first in-place signal, the motor to stop.

6. The electric stapler as claimed in claim 1, wherein when the knife pushing member performs the knife feeding action and reaches the final firing position, the control module controls the firing unit to switch to be in from an effective state to a failed state, so as to control the knife pushing member to no longer perform knife feeding; and
when the knife pushing member performs the knife feeding action and reaches the final firing position, the control module controls, when detecting the knife returning signal, the knife pushing member to leave the final firing position and perform the knife returning action towards the initial position until the initial position is reached.

7. The electric stapler as claimed in claim 1, wherein when the knife pushing member performs the knife feeding action and reaches the final firing position, the control module controls the firing unit to switch to be in from an effective state to a failed state, so as to control the knife pushing member to no longer perform knife feeding; and
when the knife pushing member performs the knife feeding action and reaches the final firing position, the control module controls, when detecting the knife returning signal, the knife pushing member to leave the final firing position and perform the knife returning action towards the initial position, and during the knife returning action, the control module controls, when detecting the knife returning stopping signal, the knife pushing member to stop the knife returning action.

8. The electric stapler as claimed in claim 7, wherein when the knife pushing member has a third state of performing the knife returning action after reaching the final firing position and stopping between the initial position and the final firing position, and in the third state, the control module controls the firing unit to be kept in a failed state, so as to control the knife pushing member to no longer perform knife feeding.

9. The electric stapler as claimed in claim 6 or 7, wherein the trigger further comprises a second position detection unit configured to detect whether the knife pushing member is located in the final firing position and to output a second in-place signal representing that the knife pushing member is located in the final firing position, and the control module controls, based on the second in-place signal, the motor to stop and controls the firing unit to switch to be in from the effective state to the failed state, and controls, during the knife returning action, the firing unit to be kept in the failed state.

10. A control method for an electric stapler, wherein the electric stapler comprises a cutting knife assembly and a motor, the cutting knife assembly comprises a knife pushing member, and the motor is configured to drive the knife pushing member to move; and the control method comprises:
controlling the motor to rotate along a first direction, so that the motor drives the knife pushing member to perform a knife feeding action towards a final firing position;
when the knife pushing member has not reached the final firing position, controlling the motor to rotate along a second direction that is opposite to the first direction, so that the motor drives the knife pushing member to perform a knife returning action towards an initial position; and
when the knife pushing member has not returned to the initial position, controlling the motor to rotate along the first direction, so that the motor drives the knife pushing member to continue performing the knife feeding action towards the final firing position.
